Europäisches Patentamt

European Patent Office

Office européen des brevets

(11) Publication number: **0 396 048**
**A1**

## (12) EUROPEAN PATENT APPLICATION

(21) Application number: **90108102.6**

(22) Date of filing: **27.04.90**

(30) Priority: **01.05.89 JP 50227/89**

(43) Date of publication of application:
**07.11.90 Bulletin 90/45**

(84) Designated Contracting States:
**CH DE FR GB IT LI NL SE**

(51) Int. Cl.⁵: **A61B 5/0408**

(72) Inventor: **Imamura, Eisaburo**
**2-23, Nishi 3-chome**
**Kunitachi-shi, Tokyo(JP)**

(71) Applicant: **MEDARD LIMITED COMPANY**
**2-23, Nishi 3-chome**
**Kunitachi-shi, Tokyo(JP)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Electrocardiographic electrode assembly.**

㊼ An electrocardiographic electrode assembly comprising electrodes (1-6) made of an electroconductive material and having hemispherically shaped lower portions (1a), an insulative sheet (20) for supporting the electrodes, and conductor connections (11-16) provided on the electrodes, which is placed on the body of a patient to record an electrocardio-gram, thereby eliminating the need for tedious preparation work such as removing of stockings and preventing disconnection of the electrodes during measurement and the occurrence of ecchymosis, which are seen with prior art nipping and sucking type electrodes.

FIG. 1

FIG. 3

# Electrocardiographic Electrode Assembly

## Background of the Invention

This invention relates to an electrode assembly which is used in recording of an electrocardiogram.

Electrocardiography is a testing method for diagnosing cardiac diseases and arrhythmia. An electrocardiogram is a record of electric current generated by the heart, picked up through electrodes which are attached to the surface of the body skin.

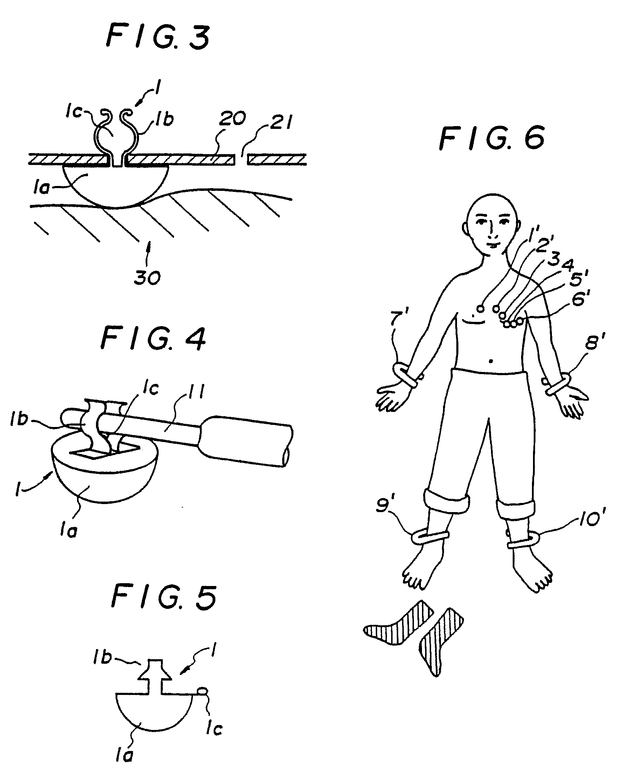

Electrocardiographic electrodes may be made of a good electrical conductor, and their sizes and shapes are not specifically limited. Heretofore, as methods to attach electrocardiographic electrodes to the skin surface, the electrodes include a nipping type and a sucking type. Fig.6 shows these prior art methods; 1'-6' are sucking type electrodes and 7'-10' are nipping type electrodes.

The nipping type electrodes are attached to four positions, on both wrists and both ankles, to record the standard induction (induction number: 3) and the limb induction (induction number: 3). However, this type of electrodes have a problem in that, since the electrode plates are required to directly contact the skin, stockings must be removed which, especially for women, is tedious and time-consuming.

The sucking type electrodes comprise suckers made of rubber, which produce a negative pressure to attract the peripheral skin, thereby fixing the electrodes to the skin. This type of electrodes are used to record the breast induction (induction number: 6). However, the sucking type electrodes tend to come off, and, if the electrodes come off during measurement, the measurement has to be interrupted. Furthermore, since the electrodes attract the peripheral skin, they tend to cause ecchymosis, which remains for several days and is thus disliked especially by women.

## Summary of the Invention

It is a primary object of the present invention to provide an electrocardiographic electrode assembly which eliminates the above prior art defects of electrocardiographic electrodes.

In accordance with the present invention which attains the above object, there is provided an electrocardiographic electrode assembly comprising electrodes made of an electroconductive material and having hemispherically shaped lower portions, an insulative sheet for supporting the electrodes, and conductor connections provided on the electrodes.

## Brief Description of the Drawings

Fig.1 and Fig.2 are schematic perspective views of the electrocardiographic electrode assembly according to the present invention.

Fig.3 is a schematic enlarged view showing part of the inventive electrocardiographic electrode assembly.

Fig.4 is a schematic perspective view showing connection between an electrode and a conductor.

Fig.5 is a schematic front view of another example of electrode used in the present invention.

Fig.6 is a schematic perspective view showing prior art electrocardiographic electrodes.

Fig.7 is an electrocardiogram recorded using the inventive electrocardiographic electrode assembly.

## Description of the Preferred Embodiments

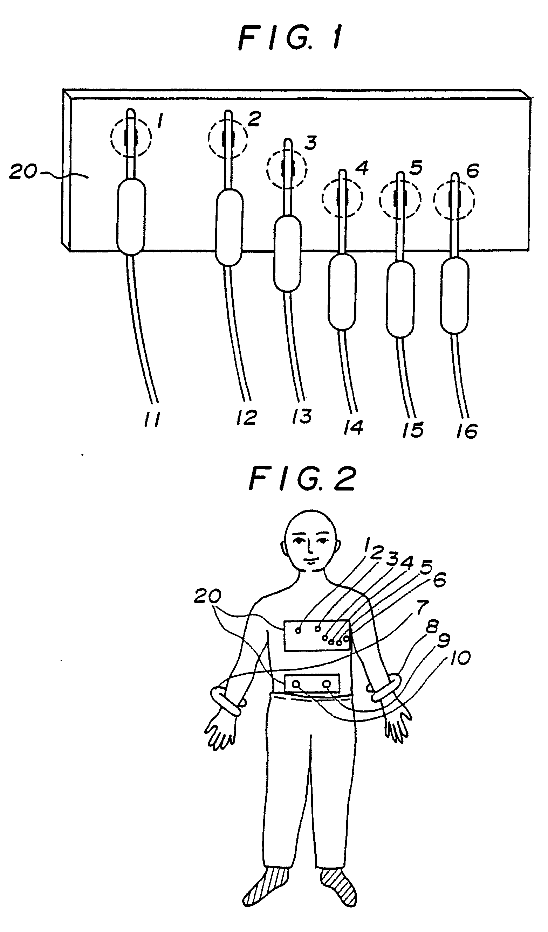

Fig.1 and Fig .2 are schematic perspective views of an embodiment of the electrocardiographic electrode assembly according to the present invention. Fig.1 shows the inventive electrode assembly used on the breast, and Fig .2 shows the inventive electrode assembly being applied to the body. Numerals 1-6 indicate portions made of an electroconductive material, of which lower portions, that is, portions to contact the body, are hemispherically shaped. Numeral 20 indicates an insulative sheet supporting the electrodes 1-6. Numerals 11-16 indicate conductors connected to the connections of the electrodes 1-6. The electrodes 1-6 are supported by the insulative sheet 20 so as to come in contact against the breast, which pick up the breast induction. Electrodes 9 and 10 shown in Fig.2 are supported on the insulative sheet 20 so as to come in contact against the right and left sides of the abdomen, which pick up the leg induction.

With the electrocardiographic electrode assembly according to the present invention, the insulative sheet with the supported electrodes shown in Fig.1 is placed on the breast of the patient, and the conductors are connected in position to an electrocardiograph to record an electrocardiogram. Fig.3 is a schematic enlarged cross sectional view showing the electrode 1 attached to the insulative sheet 20. The electrode 1 has a hemispherically shaped lower portion 1a, which comes in contact with a body 30. The electrode 1 also has an attachment portion 1b for easy attachment to the insulative sheet 20. Numeral 1c indicates a connection to connect a conductor to the electrode 1, which, in this example, forms a receptacle for the conductor. The insulative sheet 20 is provided with a plurality of holes 21 spaced apart at appropriate intervals, and the attachment portion 1b of the electrode 1 is inserted in an appropriate position according to the size of the body 30 of the patient, to be supported on the insulative sheet 20 as shown in Fig.3. Fig.4 is schematic perspective view showing the electrode 1 with a conductor 11 inserted into the receptacle 1c as the connection of the electrode 1. Fig.5 is a schematic front view showing another embodiment of the electrode used in the present invention. This electrode is attached to the insulative sheet between a protrusion of the attachment portion 1b and the hemispherical portion 1a.

The electrode with the hemispherical lower portion may be made of an electroconductive material of any type, normally of a good electroconductive material. The reason why the lower portion of the electrode is hemispherically formed is that when the insulative sheet with the electrodes is placed on the body of the patient, good contact of the electrodes with the body is assured even if the electrodes are inclined by nearly 90 degrees depending on the shape of the body. The electrode is provided at an appropriate position with a connection to connect a conductor.

The insulative sheet comprises a cloth, a plastic sheet, a rubber sheet, or a laminate thereof. The insulative sheet is preferably provided with an appropriate number of holes or cut-outs spaced apart at appropriate intervals so that the mounting portions of the electrodes can be inserted into them.

In the electrocardiographic electrode assembly according to the present invention, the electrodes are supported at appropriate positions on the insulative sheet, the insulative sheet is placed as is on the breast of the patient, and conductors from the electrodes are connected in position to an electrocardiograph to record an electrocardiogram, thereby providing easy electrocardiographic recording. Furthermore, it can eliminate the need for tedious preparation work such as removing of stockings and prevent possible disconnection of the electrodes and the occurrence of ecchymosis, which are seen with the prior art nipping and sucking type electrodes.

Moreover, in the present invention, since the lower portions of the electrodes which come in contact with the body are hemispherically shaped, when the electrodes supported on the insulative sheet are placed on the body of the patient, good contact of the electrodes with the body is assured even if the electrodes are inclined by nearly 90 degrees with respect to the portion of the body, for example, to the chest walls.

Thus, the inventive electrocardiographic electrode assembly has various advantageous effects.

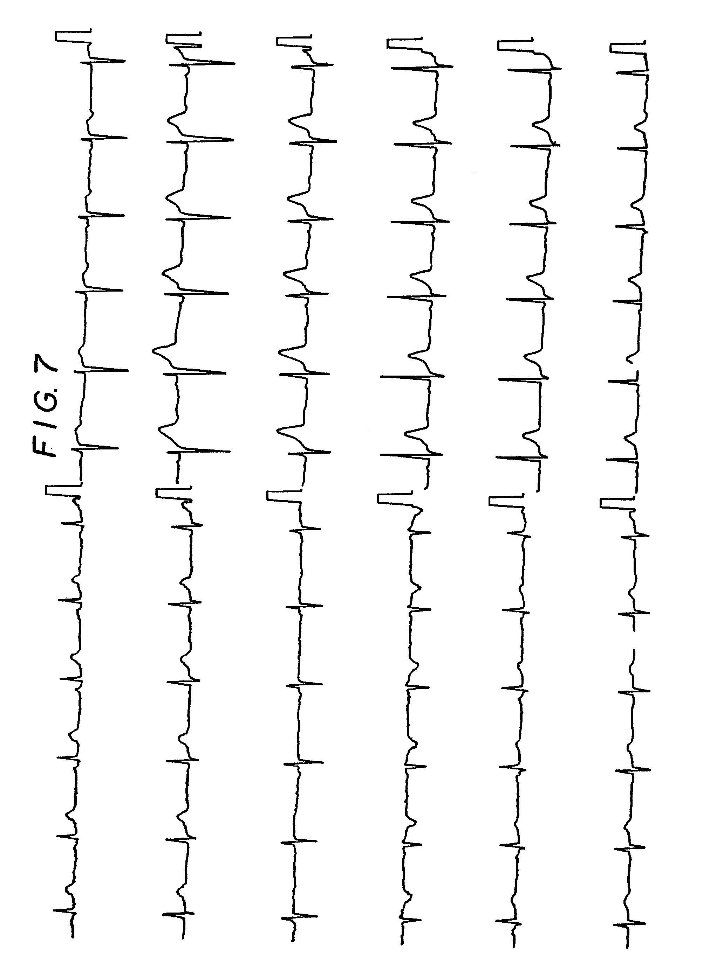

Using the inventive electrocardiographic electrode, a very clear electrocardiogram was obtained as shown in Fig. 7.

## Claims

An electrocardiographic electrode assembly comprising electrodes made of an electroconductive material and having hemispherically shaped lower portions, an insulative sheet for supporting said electrodes, and conductor connections provided on said electrodes.

# F I G. 1

# F I G. 2

FIG. 3

FIG. 6

FIG. 4

FIG. 5

F I G. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 583 549 (SAMIR MANOLI)<br>* column 4, lines 7-65; figures 2-4 * | 1 | A 61 B 5/0408 |
| X | EP-A-0 284 943 (FUKUDA DNESHI CO. LTD.)<br>* column 4, line 6 - column 6, line 27; figures 1,2,5 * | 1 | |
| A | US-A-4 233 987 (A. FEINGOLD)<br>* column 1, line 56 - column 2, line 37; figures 1-3 * | 1 | |

TECHNICAL FIELDS
SEARCHED (Int. Cl.5)

A 61 B 5/00

The present search report has been drawn up for all claims

| Place of search | Date of completion of the search | Examiner |
|---|---|---|
| BERLIN | 25-07-1990 | WEIHS J.A. |

EPO FORM 1503 03.82 (P0401)